Europäisches Patentamt

European Patent Office

Office européen des brevets

(19)

(11) **EP 1 243 230 A2**

(12) **EUROPÄISCHE PATENTANMELDUNG**

(43) Veröffentlichungstag:
 **25.09.2002 Patentblatt 2002/39**

(51) Int Cl.⁷: **A61C 9/00**, A61C 13/00,
 A61C 13/15

(21) Anmeldenummer: **02005200.7**

(22) Anmeldetag: **08.03.2002**

(84) Benannte Vertragsstaaten:
 **AT BE CH CY DE DK ES FI FR GB GR IE IT LI LU
 MC NL PT SE TR**
 Benannte Erstreckungsstaaten:
 **AL LT LV MK RO SI**

(30) Priorität: **22.03.2001 DE 10114243**

(71) Anmelder: **Heraeus Kulzer GmbH & Co.KG
 63450 Hanau (DE)**

(72) Erfinder:
 • **Stange, Frank
 61250 Usingen (DE)**

 • **Savic, Novica
 63691 Ranstadt-Dauernheim (DE)**
 • **Erdrich, Albert, Dr.
 61231 Bad Nauheim (DE)**
 • **Puchalska, Teresa
 61267 Neu-Anspach (DE)**

(74) Vertreter: **Kühn, Hans-Christian
 Heraeus Holding GmbH,
 Stabsstelle Schutzrechte,
 Heraeusstrasse 12-14
 63450 Hanau (DE)**

(54) **Verfahren zur Herstellung einer Zahnprothese sowie Prothesenwerkstoff**

(57) Verfahren zur Herstellung einer Prothese, mit:

a1) Erstellen einer Präzisionsabformung mittels einer Abdruckmasse,
b1) Erstellen eines Arbeitsmodells,
c1) Aufbringen einer Basisplatte aus einem aushärtbaren Prothesenwerkstoff und anschließende Aushärtung,
d1) Aufbringen eines Isolierfilms auf der Basisplatte,
e1) Durchführung einer Zahnaufstellung in Wachs auf der isolierten Basisplatte,
f1) äußeres Fassen von Zahnbögen unter Zuhilfenahme einer Einbettmasse,
g1) Ausbrühen der Wachsaufstellung und Entfernen des Isolierfilms,
h1) Füllen des nach dem Ausbrühen des Wachses entstandenen Hohlraums mit dem Prothesenwerkstoff und anschließende Aushärtung;

oder

a2) Erstellen einer Präzisionsabformung mittels einer Abdruckmasse,
b2) Erstellen eines Arbeitsmodells,
c2) Aufbringen einer Basisplatte aus einem lichtaushärtbarem Prothesenwerkstoff und anschließende Aushärtung,
d2) Durchführung einer Zahnaufstellung in lichtaushärtbarem Prothesenwerkstoff und anschließende Aushärtung.

EP 1 243 230 A2

**Beschreibung**

Die Erfindung betrifft ein Verfahren zur Herstellung einer Prothese, einen Prothesenwerkstoff sowie dessen Verwendungen.

Generell sind drei unterschiedliche Hauptmaterialklassen zur Herstellung von totalprothetischen Arbeiten bekannt. Dies sind auf Polymethylmethacrylat (PMMA) basierende Zweikomponentenmaterialien, PMMA-freie heißhärtende Materialien sowie thermoplastisch verarbeitbare Spritzgießmassen.

Allen diesen Werkstoffen gemeinsam sind die zur Vorbereitung der Kunststoffverarbeitung notwendigen Arbeiten.

Nach der Erstellung der Präzisionsabformung am Patienten und der Erstellung eines Arbeitsmodelles aus Gips wird mittels eines aushärtbaren Materials eine sog. Basisplatte auf das Gipsmodell aufgebracht und polymerisiert. Diese Basisplatte dient als Grundlage zur Aufstellung der späteren Prothese in Wachs und gibt ausreichende Stabilität auch für kurzzeitige Anproben im Patientenmund.

Nachteilig hierbei ist jedoch, dass diese Basisplatte vor der Umsetzung der Wachsaufstellung in Kunststoff entfernt und durch auszubrühendes Wachs ersetzt werden muss.

In einem weiteren Schritt wird die in Wachs aufgestellte Arbeit vollständig in Gips eingebettet und nach dessen Aushärtung das Wachs durch Erwärmen entfernt. In dem entstandenen Hohlraum wird nach verschiedenen Methoden der Kunststoff eingebracht und verfestigt.

Nachteilig an diesem traditionellen Verfahren sind die folgenden Punkte:

- die vollständige Einbettung unter Verwendung von Gips ist wegen der notwendigen Abbindezeit zeitaufwendig,
- wegen Unverträglichkeit zwischen dem wasserhaltigen Gips und dem Kunststoff muss ein Isoliermittel verwendet werden, wobei es immer wieder bei ungenügender Isolierung zu Verfärbungen im Kunststoff kommen kann,
- die Verwendung von lichthärtenden Materialien ist ausgeschlossen und eine Kontrolle während der Befüllung nicht möglich.

Im besonderen nachteilig ist es, dass stets der gesamte Kunststoff in einem Schritt eingebracht und polymerisiert wird, was sich wegen des Polymerisationsschrumpfes und des thermischen Schrumpfes negativ auf die Formstabilität auswirkt.

Aus dem Vorgenannten ergibt sich das Problem, insbesondere ein Verfahren zur Herstellung einer Prothese sowie einen diesbezüglichen Prothesenwerkstoff zur Verfügung zu stellen, das bzw. der die oben genannten Vorteile zumindest teilweise nicht mehr aufweist. Dies bezieht sich insbesondere auf die zu verbessernde Formstabilität.

Dieses Problem wird erfindungsgemäß durch ein Verfahren nach Anspruch 1, einen Prothesenwerkstoff nach Anspruch 8 sowie durch Verwendungen nach Anspruch 9 und 10 gelöst.

Beim erfindungsgemäßen Verfahren wird zunächst eine anatomische Abformung der Kiefer eines Patienten mittels einer üblichen Abdruckmasse, beispielsweise additionsvernetzendem Silikon oder Alginat, erstellt.

Anschließend wird ein Arbeitsmodell, insbesondere aus Gips, erstellt. Im Anschluss daran wird eine Basisplatte aus einem aushärtbaren Prothesenwerkstoff, insbesondere aus einem erfindungsgemäßen (siehe unten), aufgebracht und anschließend ausgehärtet. Dieses stellt den Kern der vorliegenden Erfindung dar, nämlich die Verwendung eines Prothesenwerkstoffes, insbesondere eines erfindungsgemäßen, als Basisplattenmaterial.

Danach wird auf der Basisplatte ein Isolierfilm aufgebracht, um anschließend eine Zahnaufstellung in Wachs auf der isolierten Basisplatte durchzuführen. Die Zahnbögen werden von außen unter Zuhilfenahme einer Einbettmasse, beispielsweise eines transparenten additionsvernetzenden Silikons oder lichthärtenden methacrylatbasierenden Einbettmaterials, gefasst. Im Anschluss daran wird die Wachsaufstellung ausgebrüht und der Isolierfilm entfernt, um daraufhin den nach dem Ausbrühen des Wachses entstandenen Hohlraum mit dem Prothesenwerkstoff zu füllen und diesen auszuhärten.

Bei einer weiteren Variante des erfindungsgemäßen Verfahrens wird zunächst eine Präzisionsabformung mittels einer Abdruckmasse erstellt, danach ein Arbeitsmodell erstellt, eine Basisplatte aus einem lichtaushärtbaren Prothesenwerkstoff aufgebracht und ausgehärtet, um schließlich eine Zahnaufstellung im lichtaushärtbarem Prothesenwerkstoff durchzuführen und anschließend auszuhärten.

Die nachfolgenden Ausgestaltungen des erfindungsgemäßen Verfahrens haben sich in der Praxis bewährt und somit als vorteilhaft herausgestellt:

Zunächst ist es von Vorteil, wenn beim letzten Verfahrensschritt der zweiten Variante die Zahnaufstellung unter Beachtung der Kieferrelationen schrittweise, insbesondere mit einem Artikulator, aufgebaut und punktuell mit Licht ausgehärtet wird, da nur dies die korrekte Position zu den Antagonistenzähnen sicherstellt und durch punktuelles Härten ein schrumpfbedingter Verzug vermieden werden kann.

Weiterhin ist es vorteilhaft, wenn nach der punktuellen Aushärtung die Prothese abschließend nochmals als Ganzes mit Licht bestrahlend ausgehärtet wird, damit ein homogener und zur Erzielung ausreichender Festigkeiten notwendiger Polymerisationsumsatz erzielt wird.

Weiterhin ist es von Vorteil, dass als Basisplattenmaterial eine Zusammensetzung verwendet wird, die aus den folgenden Bestandteilen besteht:

1. mehrfunktionelles Urethanmethacrylat/-acrylat: 5 - 46 Gewichts-%,
2. mehrfunktionelles, hochmolekulares Acrylatharz: 2,5 - 20 Gewichts-%,
3. mehrfunktioneller Reaktivverdünner: 5 - 15 Gewichts-%,
4. Bis-GMA und/oder ethoxylierte Derivate: 1 - 15 Gewichts-%,
5. Füllstoff: 2 - 15 Gewichts-%,
6. organischer Füllstoff auf Basis PMMA/Copolymer: 5 - 30 Gewichts-%,
7. Splitterpolymerisat: 0 - 30 Gewichts-%,
8. anorganisches Glas: 0 - 10 Gewichts-%,
9. Photoinitiator: 0,1 - 2 Gewichts-% und
10. Farbpigment: 0 - 1 Gewichts-%.

Bei den Verbindungen zu 1. bis 10. handelt es sich hierbei insbesondere um:

Zu 1.:
Aliphatisches Urethanacrylat/-methacrylat, Molmasse 400 - 700 g/mol, Viskosität bei 23 °C:

$$10^3 - 5 \times 10^4 \text{ mPas.}$$

Zu 2.:
Polyestertriurethanacrylat mit einer Molmasse von 900 - 1500 g/mol, Viskosität bei 60 °C,

$$10^3 - 3 \times 10^4 \text{ mPas.}$$

Zu 3.:
Allgemein mehrfunktionelle aliphatische Meth-/acrylate, Glykoldimethacrylat, z. B. Triethylenglykoldimethacrylat, alkylkettenhaltige Dimethacrylate wie Dodecandioldimethacrylat, Polyetherpolyolacrylate z. B. Pentaerythritoltetraacrylat.

Zu 4.:
Bis-GMA oder ethoxylierte Bis-GMA-Derivate.

Zu 5.:
Pyrogenes $SiO_2$, Oberfläche nach BET 50 - 250 m$^2$/g, Primärteilchengröße 7 - 40 nm, Stampfdichte: 50 - 150 g/l; kann vorteilhafterweise hydrophobiert oder mit Silanen, vorzugsweise mit Methacrylsilanen, funktionalisiert sein.

Zu 6.:
Vorteilhafterweise Primärteilchengrößen < 20 μm, wird sprühgetrocknet in agglomerierter Form geliefert.

Kern-Schale-Produkte:
Vernetzter Kern aus PMMA mit Anteilen der unter 1 und/oder 3 genannten Komponenten, Schale aus PMMA mit Anteilen von Alkylmethacrylaten, z. B. Butylmethacrylat.

Emulsionspolymerisate:
PMMA mit Anteilen Alkylmethacrylat, z. B. Isobutylmethacrylat (vernetzt mit Anteilen mehrfunktioneller Meth-/acrylate, z. B. aliphatische Dimethacrylate oder Glykoldimethacrylate).

Zu 7.:
Splitterpolymerisate
Zusammensetzung: Aliphatisches Dimethacrylat, z. B. Dodecandioldimethacrylat, pyrogenes $SiO_2$, wie unter Punkt 5. Diese Mischung wird polymerisiert, gemahlen und silanisiert und kann dann als Füllstoff verwendet wer-den; Primärteilchengröße 15 - 40 μm. Bevorzugt ist ein Anteil des Splitterpolymerisats von 5 - 30 Gewichts-%.

Zu 8.:

Anorganische Gläser

Barium-aluminiumboro-Silikate $\leq 5\ \mu m$, vorteilhaft $\leq 1\ \mu m$, vorteilhafterweise oberflächenmodifiziert, z. B. mit methacrylgruppenhaltigen Silanen.

Zu 9.:

Mögliche verwendbare Photoinitiatorsysteme

Champerchinon, Acylphophinoxid-/Bisacylphoyhinoxid-Derivate sowie Hydroxyalkylphenone, jeweils vorteilshafterweise in Kombination mit einem aminhaltigen Synergisten z. B. Alkylaminobenzoate.

Zu 10.:

Kombination aus organischem Farbpigment und Farbstoff sowie anorganischem Trübungsmittel.
Trübungsmittel: Titandioxid
Rotpigmente: organische Perylenpigmente
Blau: Anthrachinon-Farbstoff.

Als organischer Füllstoff geeignet sind ausschließlich Copolymere in Form von Perlpolymerisaten mit Vernetzeranteilen > 5 Gewichts-%, da sonst durch unerwünschte Anquellvorgänge die Viskosität über die Zeit extrem zunimmt. Besonders geeignet sind in diesem Zusammenhang sog. vernetzte Kern-Schale-Polymere, die aus einem stärker vernetzten Kern und einer schwächer bzw. nicht vernetzten Schale bestehen, wodurch ein definiertes Anquellen möglich wird. Ebenfalls besonders geeignet sind Emulsionspolymere mit definierten höher vernetzten Bereichen, die gleichfalls nur begrenzt lokal anquellen. Durch dieses partielle Anquellen werden die Füllstoffe derart in die Monomermatrix eingebunden, dass sehr hohe Schlagzähigkeiten bis ca. 8 kJ/m$^2$ erzielbar sind. (Produktbeispiel sind Perlpolymerisate, beispielsweise aus der Plexigum-Produktreihe der Firma Röhm, Darmstadt, welche sich in den Versuchen als geeignet erwiesen.)

Die Korngrößen geeigneter Polymere liegen vorteilhafterweise im Bereich $\leq 50\ \mu m$, insbesondere $\leq 20\ \mu m$.

Alternativ verwendbar sind auch siliconmodifizierte Polymere, ebenfalls auf Basis von Kern-Schale-Produkten.

Das zu verwendende Splitterpolymer besteht aus einer vorpolymerisierten Mischung aus mehrfunktionellen Meth- oder acrylaten mit anorganischen Füllstoffanteilen. Als Monomere kommen mehrfunktionale Meth-/acrylate zum Einsatz, als Füllstoffe werden pyrogene Siliciumdioxide eingesetzt. Die polymerisierten und gemahlenen Mischungen werden vor Verwendung als Füllstoff silanisiert, d. h., mit einer funktionellen methacrylathaltigen Schicht überzogen. Auf diese Weise kann eine chemische Anbindung zwischen Splitterpolymerisat und Monomermatrix des Basisplattenmaterials erzielt werden.

Die Korngrößen der einzusetzenden Splitterpolymerisate betragen in der Regel $\leq 30\ \mu m$, wobei besonders der Erhalt einer möglichst hohen Transparenz ein wichtiges Kriterium ist. Die Transparenz des Füllstoffes in Schichtstärken von 3 mm sollte bei mindestens 30 - 40 % liegen.

Optimalerweise eingesetzt werden anorganische Gläser, die üblicherweise als Zentralfüllstoff für Kompositmaterialien verwendet werden. Zu bevorzugen sind Mengen unterhalb 10 Gewichts-%, da diese anorganischen Füllstoffe zwar die mechanischen Eigenschaften verbessern, jedoch gleichzeitig auch Parameter wie Sprödigkeit, Bearbeitbarkeit und Plaqueaffinität negativ beeinflussen.

Geeignet sind beispielsweise Bariumgläser, beispielsweise Bariumaluminiumboro-Silikate, mit Korngrößen $\leq 5\ \mu m$, insbesondere $\leq 1\ \mu m$.

Die Vorteile des erfindungsgemäßen Verfahrens und des erfindungsgemäßen Prothesenkunststoffes liegen auf der Hand:
Zunächst ist es evident, dass der Wachsaufbau bereits auf der späteren Prothesenbasis erfolgt, so dass die Passgenauigkeit bei Einprobe am Patienten der Passgenauigkeit der späteren Prothese entspricht.

Weiterhin fällt der Austausch der Basisplatte gegen Wachs weg, so dass eine höhere Genauigkeit gepaart mit einer feststellbaren Zeitersparnis erzielt wird.

Häufig entfällt ein in der Praxis notwendig nachträglicher Unterfütterungsschritt vor Anfertigung der Wachsaufstellung.

Durch die mehrstufige Herstellung wird der Polymerisationsschrumpf der gesamten Prothesenarbeit verringert.

Darüber hinaus kann zumindest bei der zweiten Variante des erfindungsgemäßen Verfahrens schrittweise gearbeitet werden und durch die stückweise Polymerisierung ein Wegfließen des Materials verhindert werden.

Durch das offene Arbeiten während der Fertigstellung ist eine direkte Erfolgskontrolle des jeweiligen Arbeitsschrittes möglich.

Weiterhin ergibt sich durch die Anquellbarkeit bzw. chemische Anbindbarkeit der Füllstoffe ein homogenes Material mit hoher Festigkeit.

Durch die definierte Anquellbarkeit des Splitterpolymerisats bleibt die eingestellte Viskosität des Teiges über einen langen Zeitraum konstant.

Die hohen organischen Füllstoffanteile stellen beispielsweise in bezug auf optische Eigenschaften eine hohe Verträglichkeit zur Matrix sicher. Dadurch lassen sich die für die Verwendung als Prothesenwerkstoff notwendigen Aushärtetiefen erzielen.

Durch die nur geringen Anteile an anorganischen Füllstoffen sind vorgefertigte Platten bei Lagerung zwar noch dimensionsstabil, weisen jedoch noch nicht die Nachteile herkömmlicher Komposite bezüglich Plaqueaffinität und schlechter Polierbarkeit auf.

Somit ist eine Verwendung des erfindungsgemäßen Prothesenwerkstoffes für teilprothetische, reparatur- und kieferorthopädische Arbeiten evident.

Die Erfindung wird anhand des nachfolgenden Beispiels näher erläutert:

| Zuordnung: | Zusammensetzung: | | | |
|---|---|---|---|---|
| | Komponente | Mischung 1 | Mischung 2 | Mischung 3 |
| 1. | Urethandimethacrylat | 45 | 40 | 38,5 |
| 2. | Polyesterurethantriacrylat | 5,2 | 5 | 4,5 |
| 3. | Oligoethertetraacrylat | 6,2 | 6 | 5 |
| 3. | Trimethylolpropantrimethacrylat | 7,8 | 7,5 | 7 |
| 4. | Ethoxyliertes BIS-GMA | 3,2 | 3 | 2,8 |
| 5. | Pyrogenes Si02 | 11,1 | 14,5 | 12 |
| 9. | Photoinitiatoren/ Stabilisatoren | 0,5 | 0,5 | 0,5 |
| 10. | Farbpigmente | 0,006 | 0,006 | 0,006 |
| 6. | Vernetztes Perlpolymerisat | 21 | 17,5 | 14,7 |
| 7. | Splitterpolymerisat | - | 6 | 10 |
| 8. | Anorg. ultrafeines Glas | - | - | 5 |
| | Mechanische Festigkeit | | | |
| | Biegefestigkeit Anf. EN ISO 1567 > 60 MPa | 70 | 87 | 76 |
| | E-Modul Anf. EN ISO 1567 > 2000 MPa | 2100 | 2700 | 2500 |

**Patentansprüche**

1. Verfahren zur Herstellung einer Prothese, mit:

   a1) Erstellen einer Präzisionsabformung mittels einer Abdruckmasse,
   b1) Erstellen eines Arbeitsmodells,
   c1) Aufbringen einer Basisplatte aus einem aushärtbaren Prothesenwerkstoff und anschließende Aushärtung,
   d1) Aufbringen eines Isolierfilms auf der Basisplatte,
   e1) Durchführung einer Zahnaufstellung in Wachs auf der isolierten Basisplatte,
   f1) äußeres Fassen von Zahnbögen unter Zuhilfenahme einer Einbettmasse,
   g1) Ausbrühen der Wachsaufstellung und Entfernen des Isolierfilms,
   h1) Füllen des nach dem Ausbrühen des Wachses entstandenen Hohlraums mit dem Prothesenwerkstoff und anschließende Aushärtung;
   oder
   a2) Erstellen einer Präzisionsabformung mittels einer Abdruckmasse,
   b2) Erstellen eines Arbeitsmodells,
   c2) Aufbringen einer Basisplatte aus einem lichtaushärtbarem Prothesenwerkstoff und anschließende Aushärtung,
   d2) Durchführung einer Zahnaufstellung in lichtaushärtbarem Prothesenwerkstoff und anschließende Aushärtung.

**2.** Verfahren nach Anspruch 1, **dadurch gekennzeichnet, dass** beim Verfahrensschritt d2) die Zahnaufstellung unter Beachtung der Kieferrelationen schrittweise aufgebaut und punktuell mit Licht ausgehärtet wird.

**3.** Verfahren nach Anspruch 2, **dadurch gekennzeichnet, dass** nach der punktuellen Aushärtung die Prothese abschließend nochmals als Ganzes mit Licht bestrahlend ausgehärtet wird.

**4.** Verfahren nach einem der Ansprüche 1 bis 3, **dadurch gekennzeichnet, dass** als Basisplattenmaterial eine Zusammensetzung verwendet wird, die aus den folgenden Bestandteilen besteht::

       1. mehrfunktionelles Urethanmethacrylat/-acrylat: 5 - 46 Gewichts-%,
       2. mehrfunktionelles, hochmolekulares Acrylatharz: 2,5 - 20 Gewichts-%,
       3. mehrfunktioneller Reaktivverdünner: 5 - 15 Gewichts-%,
       4. Bis-GMA und/oder ethoxylierte Derivate: 1 - 15 Gewichts-%,
       5. Füllstoff: 2 - 15 Gewichts-%,
       6. organischer Füllstoff auf Basis PMMA/Copolymer: 5 - 30 Gewichts-%,
       7. Splitterpolymerisat: 0 - 30 Gewichts-%,
       8. anorganisches Glas: 0 - 10 Gewichts-%,
       9. Photoinitiator: 0,1 - 2 Gewichts-% und
       10. Farbpigment: 0 - 1 Gewichts-%.

**5.** Verfahren nach Anspruch 4, **dadurch gekennzeichnet, dass** als organischer Füllstoff vernetzte Kern-Schale-Polymere oder vernetzte Emulsionspolymerisate eingesetzt werden.

**6.** Verfahren nach einem der Ansprüche 4 bis 5, **dadurch gekennzeichnet, dass** der Korngrö-ßendurchmesser des organischen Füllstoffs ≤ 50 µm, vorzugsweise ≤20 µm, beträgt.

**7.** Verfahren nach einem der Ansprüche 4 bis 6, **dadurch gekennzeichnet, dass** der Korngrö-ßendurchmesser des anorganischen Glases ≤ 5 um, vorzugsweise ≤ 1 µm, beträgt.

**8.** Prothesenwerkstoff, **dadurch gekennzeichnet, dass** dieser eine Zusammensetzung nach einem der Ansprüche 4 bis 7 aufweist.

**9.** Verwendung eines Prothesenwerkstoffes nach Anspruch 8 als Basisplattenmaterial in einem Verfahren nach einem der Ansprüche 1 bis 7.

**10.** Verwendung eines Prothesenwerkstoffes nach Anspruch 8 für total-/teilprothetische, reparatur- und kieferorthopädische Arbeiten.